# EUROPEAN PATENT APPLICATION

(11) **EP 1 674 580 A1**
(43) Date of publication of application: **28.06.2006**
(21) Application number: 04106965.9
(22) Date of filing: 23.12.2004
(51) Int. Cl.: C12Q 1/37, C07K 5/00

(54) **Method for identifying activators and/or inhibitors of enzyme activity**

(71) Applicant: F. HOFFMANN-LA ROCHE AG, 4070 Basel (CH)
(72) Inventor: Adessi, Céline, 68330 Huningue (FR); Enderle, Thilo, 79618 Rheinfelden (DE); Grueninger, Fiona, 4144 Arlesheim (CH); Roth, Doris, 4051 Basel (CH)
(74) Representative: Witte, Hubert

(57) **Abstract**

A method for identifying activators or inhibitors of enzyme activity comprising contacting a enzyme with (i) a compound of formula F-P-Q, wherein P represents a peptide comprising at least one cleavage site of the enzyme, wherein F represents a fluorophore which is attached to at least one amino acid residue of P, wherein Q represents a compound being able to statically quench the fluorophore F, and being attached to at least to one amino acid residue of P and, whereby F and Q are attached to amino acid residues on different sites of the enzyme cleavage site of P, and (ii) a compound of interest, and measuring the resulting fluorescent emission.

SEQUENCE LISTING

<110> F. Hoffmann-La Roche AG

<120> Method for identifying activators and/or inhibitors of enzyme activity

<130> 22878

<160> 3

<170> PatentIn version 3.2

<210> 1
<211> 42
<212> PRT
<213> Homo sapiens

<220>
<221> Abeta(1-42)
<222> (1)..(42)
<223> Accession number: 1IYT_A

<400> 1

<210> 2
<211> 8
<212> PRT
<213> Homo sapiens

<220>
<221> Fragment of Abeta(1-42)
<222> (1)..(8)

<400> 2

Lys Leu Val Phe Phe Ala Glu Asp
1               5

<210> 3
<211> 4
<212> PRT
<213> Unknown

<220>
<223> derived from N-Succinyl-Ala-Ala-Pro-Phe-p-Nitroanilide (S-7388)

<220>
<221>  Chymase substrate
<222>  (1)..(4)

<400>  3

Ala Ala Pro Phe
1

## Description

The present invention relates to a method for identifying activators or inhibitors of enzyme activity based on the statically quenching of a fluorophore by a quencher, to compounds of the formula Fluorophore-Peptide-Quencher, to activators and/or inhibitors identified by said method and pharmaceutical compositions.

There are known protease assays using the quenching effect for identifying activators or inhibitors of the enzyme activity. These assays use either collisional quenching or FRET Both, collisional quenching and FRET, have the disadvantage of creating false positives. These artifacts have to be corrected in troublesome labour.

Thus, there is a requirement in the art for rapid and sensitive methods for the identifying of enzyme activators or inhibitors

The present invention relates to a method for identifying activators or inhibitors of enzyme activity comprising
a) contacting a enzyme with (i) a compound of formula F - P - Q
   - wherein P represents a peptide comprising at least one cleavage site of the enzyme
   - wherein F represents a fluorophore which is attached to at least one amino acid residue of P
   - wherein Q represents a compound being able to statically quench the fluorophore F, and being attached to at least one amino acid residue of P and
   - whereby F and Q are attached to amino acid residues on opposite sides of the enzyme cleavage site of P
      and (ii) a compound of interest,
b) measuring the fluorescence emission, and
c) comparing the fluorescence emission of b) with a control fluorescence emission.

A control fluorescence emission may be fluorescence measurable in the absence of the candidate of interest.

The enzyme useable for the invention is a protease. Preferably the enzyme is chymase or an amyloid degrading enzyme, more preferably the enzyme is able to cleave Abeta(1-42), the most preferred enzymes are insulin degrading enzyme (IDE) and neprilysin (NEP).

The invention further relates to a compound of formula F - P - Q, wherein P represents a peptide comprising at least one enzyme cleavage site, wherein F represents a fluorophore being attached to at least one amino acid residue of P, wherein Q represents a compound being able to quench the fluorophore F statically and being attached to at least one amino acid residue of P and whereby F and Q are attached to amino acid residues on opposite sides of the enzyme cleavage site of P. Preferably, the fluorophore and the quencher are attached to opposite termini of the peptide.

Between the fluorophore and the peptide, and the peptide and the quencher may be a linker. The term "linker" as used herein refers to one or more small molecules which are used to attach the fluorophore or the quencher to at least one amino acid residue of P. The linker may be for example one or more amino acids. Further examples may be found in table 1. A preferred linker is cysteine or lysine or another molecule comprising a sulfhydryl-group or a primary amine, respectively. The fluorophore is preferably attached to the cysteine linker by a maleimide or attached to the lysine by a NHS ester.

**Table 1 gives some examples for linker. A person skilled in the art knows further such groups from the synthetic chemistry for conjugates.**

| Activated group | Linkage with | Product |
|---|---|---|
| NHS ester | amines | amide |
| isothiocyanate | amines | thiourea |
| mixed anhydride | amines | amide |
| maleimide | thiol | thioether |
| thiol | maleimide | thioethers |
| haloacetyl | thiol | thioethers |
| hydrazine | aldehyde | hydrazones |
| amines | aldehyde | amine (after reduction) |
| amines | reactive carboxylic acid | amides |

The peptide P has at least one cleavage site at which the enzyme may cut P. The length of P is one amino acid to more than 100 amino acids. Preferably, the peptide has 1 to 50 amino acids. More preferably, the peptide has 4 to 20 amino acids. Most preferably, the peptide has 4 to 10 amino acids.

P may be a fragment of a polypeptide. In this case, the fragment is preferably chosen from a region of the polypeptide with cleavage sites at which the polypeptide is often cut by the enzyme.

P may be a naturally occurring peptide, a recombinant peptide or a chemically synthesized peptide. The term "naturally occurring peptide" as used herein refers to a peptide isolated from an organism producing said peptide by nature.

The term "recombinant peptide" as used herein refers to a peptide which was produced by inserting the DNA sequence encoding the peptide in a host and expressing the peptide by that host. The methods of DNA recombinant technology for producing the recombinant peptides are well known, i.e. from Maniatis et al. in "Molecular Cloning - A laboratory Manual", Cold Spring Harbor Laboratory, 1982.

Peptides can be chemically synthesized using standard methods known in the art, preferably solid state methods such as the methods of Merrifield (*J. Am. Chem. Soc.* (1963), 85: 2149-2154) or by Houghten et al. ("Human beta-endorphin: synthesis and characterization of analogs iodinated and tritiated at tyrosine residues 1 and 27." *Int J Pept Protein Res*. (1980), 16(4):311-20 and "Chemical synthesis of an octadecapeptide with the biological and immunological properties of human heat-stable Escherichia coli enterotoxin." *Eur J Biochem.* (1984), 15;145(1):157-62.)

Preferred peptides are amyloid peptides and fragments thereof. Suitable fragments are preferably selected from regions with cleavage sites at which the peptides are often cut by the enzyme. The peptide more preferred is Abeta(1-42) (SEQ. ID NO: 1) or a fragment thereof (i.e. SEQ. ID NO: 2). The peptide most preferred has the peptide sequence Lys-Leu-Val-Phe-Phe-Ala-Glu-Asp (SEQ. ID NO: 2). Also preferred is a peptide of the peptide sequence of the formula Ala-Ala-Pro-Phe (SEQ. ID. NO: 3).

The fluorophore F is a molecule emitting fluorescence when excited with a specific wave length and being statically quenchable. Preferably, the fluorophore is able to form a non-fluorescence ground complex with the quencher. A fluorophore may be for example ATTO 590, ATTO 655, ATTO 680 (Atto Bioscience, 15010 Broschart Road, Rockville, MD 20850, USA). A fluorophore may also be functional oxazine derivatives capable of coupling of the general formula I 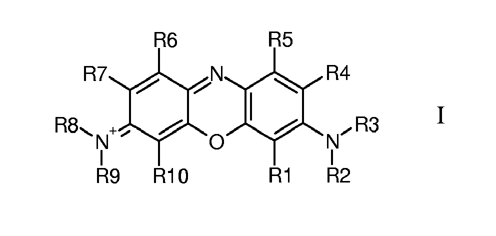 in which R₁, R₄, R₅, R₆, R₇, R₁₀ represent hydrogen, alkyl, alkoxy, hydroxy, halogen, carboxyl, sulfonyl or amino and R₂, R₃ denote hydrogen, alkyl, alkoxy, polyoxyhydrocarbyl units, phenyl, phenylalkyl which can be substituted by hydroxy, halogen, sulfonyl, carboxy, amino, alkoxycarbonyl, in which R₂ with R₁ or R₃ with R₄ can form a saturated or unsaturated C2 or C3 bridge or R2 with R3 can form a saturated or unsaturated C4 or C5 bridge and R₈, R₉ denote hydrogen, alkyl, alkoxy, polyoxyhydrocarbyl units, phenyl, phenylalkyl which can be substituted by hydroxy, halogen, sulfonyl, carboxy, amino, alkoxycarbonyl in which R₈ with R₇ or R₉ with R₁₀ can form a saturated or unsaturated C2 or C3 bridge or R₈ with R₉ can form a saturated or unsaturated C4 or C5 bridge and in which at least one of the residues R₂, R₃, R₈ or R₉ represents a non-bridge forming residue which is substituted by an activated group capable of coupling or by a group that can be activated to couple, and in which at least one of the residues R₂, R₃, R₈ or R₉ represents a bridge-forming residue which optionally can be substituted by alkyl.

It is preferred when R₃ with R₄ and/or R₇ with R₈ forms a saturated or unsaturated C3 bridge.

It is particularly preferred when R₃ with R₄ and/or R₇ with R₈ form a C3 bridge while R₂ and/or R₉ represent non-bridge forming substituents, preferably alkyl and at least one non-bridge forming substituent is substituted with an activated group capable of coupling or with a group that can be activated.

The term "polyoxyhydrocarbyl units" is understood within the sense of the present invention as polymeric or oligomeric organic residues which are linked together by O bridges. In particular this term is understood as polyethers, polyoles, soluble carbohydrates, derivatives thereof or water-soluble polymers. Polyethyleneoxy groups are particularly preferred whose size is such that the molecular weight of the total compound is 800-1200, preferably about 1000. The aforementioned polyethyleneoxy groups improve the solubility, reduce the unspecific binding of the compounds to proteins and prevent a dimerization.

An alkyl group has 1-10, preferably 1-7 carbon atoms and can be branched or straight-chained; it especially preferably has 1 to 4 carbon atoms and is in particular for example methyl, ethyl, propyl, iso-propyl, butyl, iso-butyl or tert. butyl.

Aphenylalkyl group with preferably 1-3 carbon atoms in the alkyl group is in particular a phenethyl or benzyl group.

Halogen is understood as fluorine, chlorine, bromine or iodine and preferably chlorine.

An alkoxy group in an alkoxycarbonyl group has 1-10, preferably 1-4 and especially preferably 1 or 2 C atoms.

A preferred fluorophore is the oxazin derivate of the general formula I, in which R1, R5, R6, R10 represent hydrogen, R2 represent alkyl (C2H5, Ethyl), R3 with R4 form a saturated C3 bridge (C3H6), R7 with R8 form a saturated C3 bridge (C3H6), R9 represent carboxyl (C4H6O2) (MR121, see formula II). 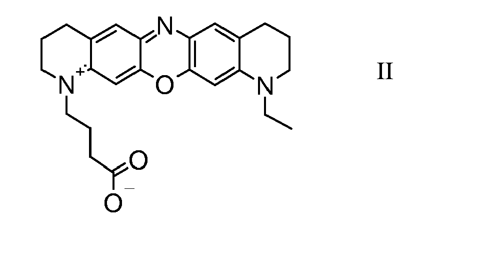 Another preferred fluorophore is the oxazin derivate of the general formula I, wherein R1, R5, R6, R10 represent hydrogen, R2 represent sulfonyl (C4H8SO3), R3 with R4 form a saturated C3 bridge (C3H6), R7 with R8 form a saturated C3 bridge (C3H6), R9 represent carboxyl (C4H6O2) (JA314, see formula III). 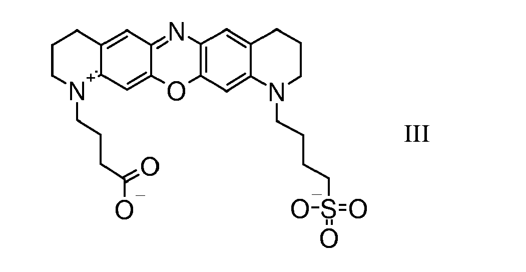 Another preferred fluorophore is the oxazin derivate of the general formula I, wherein R1, R4, R5, R6, R7, R10 represent hydrogen, R2 represent Carboxyl (C3H5O2), R3 represent Alkyl (CH3, Methyl), R8 represent alkyl (C2H5, Ethyl), R9 represent sulfonyl (C3H6SO3) (Evoblue30, see formula IV).

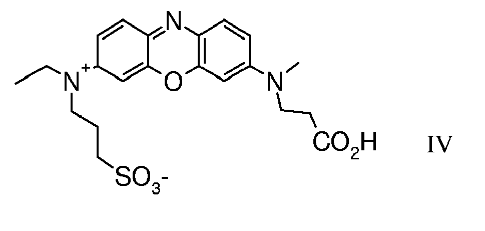

In the compounds of formula I at least one of the residues R₂, R₃, R₈, R₉ is preferab present as a non-bridge forming residue which is substituted with a linker. Preferably, the linker is an activated group capable of coupling or with a group which can be particular derived from an activatable carboxylic acid or sulfonic acid group such as for example an acid ester, an acid anhydride, an acid halogenide, preferably bromide in particular chloride or an N-hydroxysuccinimide ester. A linker compound such as DADOO (2,2-ethylendioxy)-bis(ethylamine)) maybe inserted between the activated group and the non-bridge forming residue. Preferably, the linker is maleimide.

The oxazin derivates of formula I are described in detail in EP 747 447, which is herewith specifically enclosed as reference.

The fluorophore is attached to at least one amino acid residue of P. Preferably, F is attached to the N-terminus or to the C-terminus of P. When the fluorophore is attached to the N-terminus of the peptide then the quencher Q is preferably attached to the C-terminus of the peptide P. When the fluorophore F is attached to the C-terminus of P then Q is preferably attached to the N-terminus of P.

The quencher Q reduces the fluorescence of the fluorophore by statically quenching forming a non-fluorescence ground state complex with the fluorophore (Neuweiler et al., J. Am. Chem. Soc. (2003), 125: 5324-5330). A preferred quencher is the amino acid Tryptophan. Q is attached to at least one amino acid residue of P. Preferably, Q is attached to the N-terminus or to the C-terminus of P. When Q is attached to the N-terminus of P then F is preferably attached to the C-terminus of P. When Q is attached to the C-terminus of P then F is preferably attached to the N-terminus of P.

A preferred compound is MR121-linker-Lys-Leu-Val-Phe-Phe-Ala-Glu-Asp-Trp whereby the linker is maleimide and cysteine.

The compound of formula F-P-Q may be used as substrate in the above described method for identifying activators and/or inhibitors of enzyme activity.

The term "activator" as used herein refers to a molecule that increases the activity of an enzyme. The term "activity of an enzyme" as used herein refers to the turnover rate of an enzyme for its substrate.

The term "inhibitor" as used herein refers to a molecule that decreases or stops the activity of an enzyme.

The terms "quench" or "quenching" as used herein refers to a decrease of the intensity of the fluorescence emission.

The term "static quenching" or "statically quenching" as used herein refers to quenching without a change of the life time of the fluorescence emission. Static quenching is characterized by the formation of a non-fluorescence ground state complex of the fluorophore and the quencher.

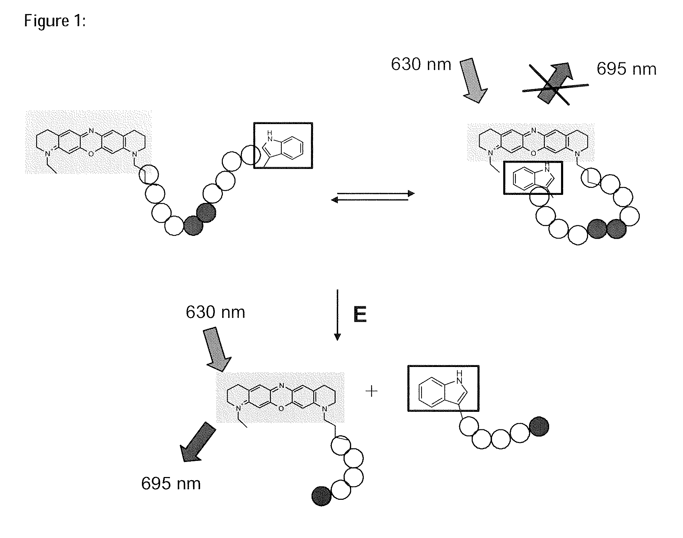

The assay principle is shown in figure 1. The uncleaved substrate, the compound of formula F-P-Q, shows no or low fluorescence when exposed to excitatory light emission because Q perturbs F. When the enzyme cleaves the peptide, F and Q are separated and Q is no longer able to reduce the fluorescence of F. Therefore there is a detectable increase of the fluorescence.

In the assay of the invention the enzyme is contacted with the substrate of formula F-P-Q and a candidate compound. If this candidate compound is an activator the amount of cleaved peptide will increase detectable in an increase of fluorescence. If the candidate compound is an inhibitor the amount of cleaved peptides will decrease detectable in a decrease of fluorescence. If the candidate is neither activator nor inhibitor there will be no change of fluorescence.

A preferred embodiment is an assay based on statically quenching of MR121 by tryptophan. While the free MR121 is a fluorophore with a high quantum efficiency, it forms a non-fluorescent ground state complex with tryptophan (Neuweiler, H.; Schulz, A.; Vaiana, A C.; Smith, J. C.; Kaul, S.; Wolfurm, J.; Sauer, M. *Angewandte Chemie* (2002), 114: 4964-4968; Neuweiler, H.; Schulz, A.; Böhmer, M.; Enderlein, J.; Sauer, M. *JACS* (2003), 125: 5324-5330; Wolfurm, J.; Sauer, M. In *WO 03*/*014742 A2:* Germany, 2001). A substrate peptide is labeled at the N-terminus with the fluorophore MR121 and at the C-terminus with tryptophan or vice versa. For the intact substrate the MR121 fluorescence is mostly quenched, if the substrate is cleaved by the enzyme the MR121 fluorescence is dequenched (Fig.1). The enzymatic reaction can be followed in a kinetic measurement detecting an increase of MR121 fluorescence during the reaction time. Calculating the slope in the linear range of the kinetic provides a robust value for the activity of a protease.

This assay is suitable for both, activator screens which are used to look for compounds increasing enzymatic activity and thus the slope as well as for inhibitor screens, looking for compounds which decrease the activity of the protease.

The invention also relates to an activator or/and inhibitor identified by the method described above. In particular, the invention provides Suramin as an activator of IDE.

The activators and inhibitors identified by the above described method may be used for the preparation of a medicament for treatment of a disease. Therefore, the present invention also relates to the use of Suramin for preparation of a medicament for treating Alzheimer disease.

The invention also pertains to a pharmaceutical composition comprising a soluble activator or inhibitor as described above, preferably Suramin, and a compatible pharmaceutically acceptable carrier material.

The carrier material can be an organic or inorganic one suitable for enteral, percutaneous or parenteral administration. Suitable carries include water, gelatin, gum arabic, lactose, starch, magnesium stearate, talc, vegetable oils, polyalkylene-glycols, petroleum jelly and the like. Furthermore, the pharmaceutical preparations may contain other pharmaceutically active agents. Additional additives such as flavouring agents, preservatives, stabilizers, emulsifying agents, buffers and the like may be added in accordance with accepted practices of pharmaceutical compounding.

The pharmaceutical preparations can be made up in any conventional form including: a) a solid form for oral administration such as tablets, capsules, pills, powders, granules and the like; b) a liquid form for oral administration such as solutions, syrups, suspensions, elixirs and the like; c) preparations for parenteral administration such as sterile solutions, suspensions or emulsions. The pharmaceutical preparations may be sterilized and/or may contain adjuvants such as preservatives, stabilizers, wetting agents, emulsifiers, salts for varying the osmotic pressure and/or buffers.

Parenteral dosage forms may be infusions or injectable solutions which can be injected intravenously or intramuscularly. These preparations can also contain other medicinally active substances. Additional additives such as preservatives, stabilizers, emulsifying agents, buffers and the like may be added in accordance with accepted practices of pharmaceutical compounding.

Having the invention now generally described, the same will become better understood by reference to the specific examples, which are included herein for purpose of illustration only and are not intended to be limiting unless otherwise specified, in connection with the following figures:

### Figures

Figure 1 shows the assay principle. The substrate, the compound of formula F-P-Q, comprises a fluorophore (grey box), a peptide (circles) with at least one cleavage site of the enzyme (filled circles) and a quencher (framed). The uncleaved substrates shows no or low fluorescence as the quencher reduces the fluorescence of the fiuorophore by collisional quenching. When the enzyme E cleaves the peptide the perturbation of the fiuorophore stops and the fluorescence increases.

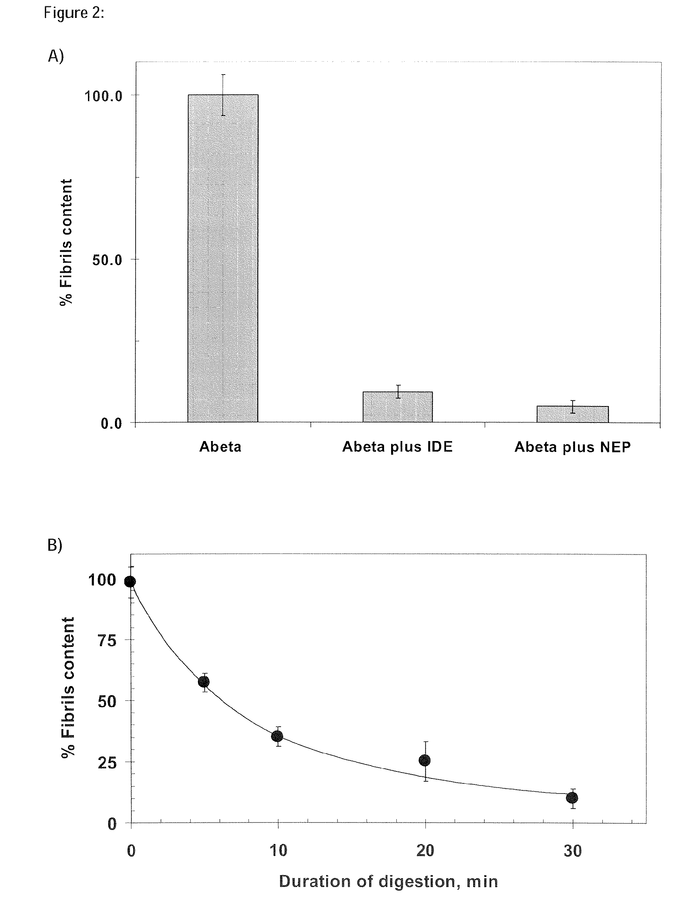

Figure 2 shows a diagram of the formation of Abeta(1-42) fibrils without enzymes, in the presence of NEP (neprilysin) and in presence with IDE (insulin degrading enzyme) evalutated by a thioflavine T assay.
   Thioflavine T assay: Abetal-42 (10 µM) alone or in the presence of NEP or IDE were incubated at 37°C for 72 h. Thioflavine T fluorescent signal indicates the ability of Abetal-42 and fragments to form fibrils (A). The degradation of Abeta 1-42 by IDE was followed as a function of time. At various time points (0-30 min) the enzymatic inhibitor, N-ethyl maleimide (NEM from Acros) was added at 1 mM final concentration. Each sample was incubated at 37°C for additional 72 hrs. Thioflavine T fluorescence measurements were performed as described above (B).

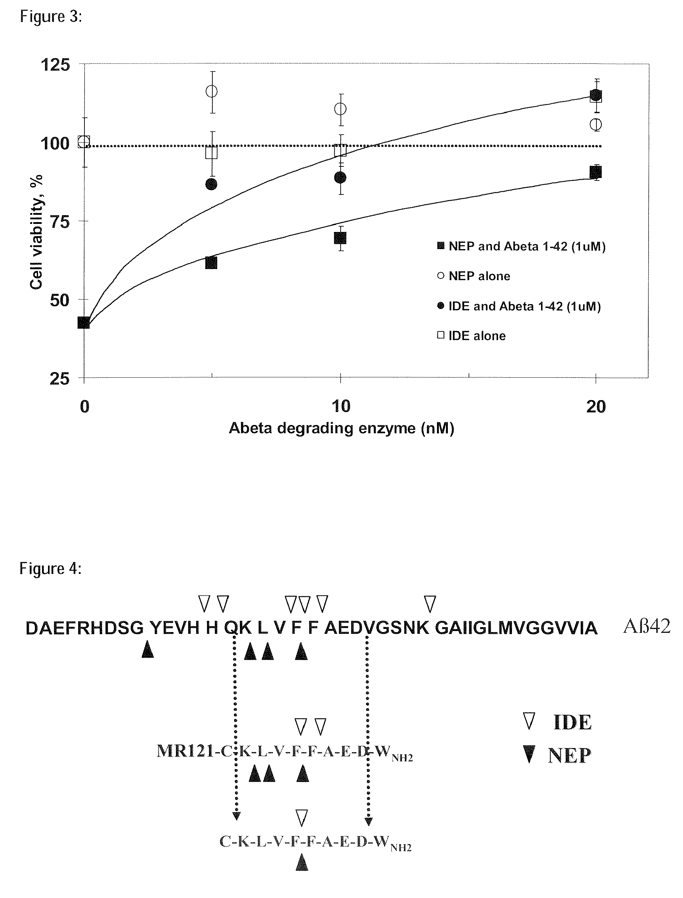

Figure 3 shows a diagram of the cell toxicity of the Abeta(1-42) fibrils. When incubated with NEP (neprilysin) or IDE (insulin degrading enzyme) the cell toxicity (MTT assay) is abolished.
   MTT assay: Abetal-42 (1 µM) alone or with NEP or IDE (10 nM) were incubated on PC12 cells (16 h). Using the MTT kit assay the cell viability was measured as a function of concentration of enzyme (nM). The black squares indicate NEP and Abeta(1-42) (1uM), the white circles indicate NEP alone, black circles indicate IDE and Abeta(1-42) (1uM), the white squares indicate IDE alone.
Figure 4 shows schematically cleavage sites of Abeta(1-42) for NEP (neprilysin) and IDE (insulin degrading enzyme). The position of the major cleavage sites of Abeta(1-42) are Cys-Abeta 16-23-Trp and MR121-Cys-Abeta 16-23-Trp identified by MS following IDE (empty arrowheads) or NEP (filled arrowheads) digestion.
Figure 5 shows a diagram of the kinetic measurements with (a) 10 nM IDE and 10 µM substrate (ratio labeled to unlabeled substrate: 1:1000), (b) 1 nM NEP and 3 µM substrate (ratio labeled to unlabeled substrate: 1:1000), and (c) 5 nM Chymase and 20 µM substrate (ratio labeled to unlabeled substrate: 1:100), crosses: substrate and enzyme, circles: only substrate.
Figure 6 shows a diagram of the Kₘ and Vₘₐₓ determination for IDE (a) and Chymase (b). In the diagram the slopes of the linear part of kinetic measurements were plotted against the total substrate concentration.
Figure 7 shows a screen of a small library. The dashed lines indicate the three standard deviation from the average signal expressed in the percent activation of the enzyme. One positive was identified: Suramin.
Figure 8 shows a diagram of the dose response curve for inhibitor A with 1 µM substrate with (filled diamonds) and without (open diamonds) 5 nM Chymase, IC₅₀=157 nM.

### Examples

Commercially available reagents referred to in the examples were used according to manufacturer's instructions unless otherwise indicated.

### Example 1: activators for degradation of Abeta (1-42)

In this example the described method is used to identify enhancers that selectively activate IDE (Insulin-degrading enzyme) or NEP (neprilysin). By enhancement of these amyloid degrading enzymes the degradation of Abeta shall be increased and thereby prevent the formation of amyloid plaques or reduce the already formed ones.

### A) Substrate

### Materials

Synthetic Amyloid beta peptide 1-42 (Abetal-42) was purchased from Bachem (Bubendorf, Switzerland);

Hexafluoro-2-Propanol was purchased from Sigma (HFIP; Sigma H-8508).

Buffer 1 (50 mM Hepes buffer pH 7.4, 100 mM NaCl, 0.05% bovine serum albumin) (Leissring MA, Lu A, Condron MM, Teplow DB, Stein RL, Farris W, Selkoe DJ Kinetics of amyloid beta-protein degradation determined by novel fluorescence- and fluorescence polarization-based assays. *J Biol Chem*. (2003), 278(39):37314-20).

### Methods

### Identification of proteolytic cleavage site of Abetal-42

Abeta synthetic peptide was pre-solubilized in HFIP as previously described in Dahlgren, K N., Manelli, A M., Stine, W. B., Jr., Baker, L. K., Krafft, G. A., and LaDu, M. J. (2002) Oligomeric and fibrillar species of amyloid-beta peptides differentially affect cell viability. *J Biol Chem*, 277: 32046-32053). HFIP pre-treated Abeta was solubilized to 10µM final concentration in Buffer 1 and incubated at room temperature either with 10n M NEP or IDE. At various time points (after 0, 2 and 5 min) the reaction mixture was removed and analyzed by HPLC and MS.

For MS analysis the solution was mixed with a saturated alpha-cyano cinnamic acid (Sigma) and applied to MALDI-target (Bruker, Bremen, Germany). The samples were analyzed with a Bruker Daltonics Ultraflex TOF/TOF mass spectrometer (Bruker, Bremen, Germany).

High performance liquid chromatography (HPLC) analysis was performed on a 1110 Agilent system using reverse phase column (Poroshell 300SB-C8 available at Agilent, 395 Page Mill Road, Palo Alto, CA 94306) and a linear gradient (20-60 % acetonitril, 01% TFA in water) over 60min. Peptides were monitored by UV light absorbance (at 205 nm and 280 nm) and peaks collected for further MS analysis. The UV area of peaks was correlated to the relative amount of each proteolytic fragment.

### Cell toxicity assay

Abetal-42 prepared at 50 microM in a 10 mM Tris buffer, pH 8 was added to the medium of PC12 cells (ATTC: CRL1721) (HAM's F-12K medium from Biosources International, Napa, California 94558, United States) containing 10% HS, 5% FCS) at 1 µM alone, or with NEP or IDE. The cells were incubated 16 h; the cellular viability was evaluated using the 3-(4,5-dimethylthiazol-2-yl)-2,5-diphenyltetrazolium bromide cell proliferation kit (MTT, Roche Molecular Biochemicals, Switzerland).

### Thioflavin T assay

Amyloid formation was quantitatively evaluated by the fluorescence emission of thioflavin T bound to amyloid fibrils. Abetal-42 at 10 microM was incubated alone, or with NEP (200 nM) or IDE (100 nM) at 37°C for 72 h. After incubation 50 mM glycine buffer (pH9.2) and 2 microM Thioflavin T were added and fluorescence measured (ex: 435 nm, em: 485 nm) in a 96 well plate reader (Polarstar galaxy BMG Fluostar OPTIMA Labtechnologies, In der Spöck 10, Offenburg, DE).
The degradation of Abeta(1-42) by IDE was stopped at various time points with the inhibitor N-ethyl maleimide (NEM from Acros Organics B.V.B.A., Janssen Pharmaceuticallaan 3A, Geel, Belgium). Abetal-42 at 10 microM was incubated alone, or with IDE (100 nM) at 37°C. At various time points (0-30 min) the enzymatic inhibitor, N-ethyl maleimide was added at 1 mM final concentration. Each sample was incubated at 37°C for additional 72 hrs. Thioflavine T fluorescence measurements were performed as described above.

### Results

### Identification of major proteolytic cleavage sites of Abetal-42.

The major cleavage site of Abetal-42 by NEP and IDE were determined by mass spectrometry and HPLC analysis (Table1). Both enzymes degraded Abetal-42 at various cleavage sites located mainly within the central hydrophobic region. IDE and NEP primarily initiated proteolysis from residues 18 to 21 and 16 to 20 respectively. Both enzymes shared a common cleavage site between the Phe19-Phe20 residues.

### NEP or IDE endopeptidase activity prevent Abeta fibril formation

The central region of Abeta is highly hydrophobic and is essential for the amyloidogenic property of the peptide. Interestingly both enzymes degrade Abeta within this central region. Both enzymes were able to abolish formation of Abetal-42 fibrils as shown in the thioflavine T assay (figure 2). Co-incubation of Abetal-42 with NEP or IDE abolished toxicity of PC12 cells induced by Abetal-42 fibrils (figure 3). These results suggested that the proteolytic activity of both enzymes was sufficient to prevent Abetal-42 aggregation and Abeta 1-42 induced cytotoxicity.

### MR121-substrate design

The substrate for the assay for identifying activators of IDE and NEP contains the main proteolytic cleavage sites identified in the central hydrophobic region of Abetal-42, namely the sequence of Abeta16-23 (Lys-Leu-Val-Phe-Phe-Ala-Glu-Asp). A cysteine was added at the N-terminus of the peptide to link the MR121 fluorophore and a tryptophan residue at the C-terminus of the peptide (see Materials and Methods).

The proteolytic fragments of MR121-C-Abeta 16-23-W and unlabeled substrate (C-Abeta 16-23.-W) following NEP and IDE digestion were identified by mass spectrometry MS and HPLC as previously described. Results confirmed that both substrates labelled and unlabeled share identical cleavage sites than those identified with Abetal-42 (Figure 4).

### B) Assay

### Material and Methods

### IDE/NEP

The 10 amino acid peptide Cys- Lys-Leu-Val-Phe-Phe-Ala-Glu-Asp-Trp contained in the substrated was synthesized with a purity of 95% by Biosyntan GmbH, Berlin, DE (For improved yield of synthesis the carboxyl group of the tryptophan was replaced by an amide group (CO-NH2)). The reactive form of the fluorophore, MR121-maleimide, is described in EP 747 447. MR121-maleimide was attached to the sulfhydryl group of the cysteine residue of the substrate peptide by covalent coupling (Derek G. Smyth, Atsuo Nagamatsu, Joseph S. Fruton; Some Reactions of N-Ethylmaleimide; *J. Am. Chem. Soc.* (1960); 82(17): 4600-4604) and purified on an analytical HPLC (Merck Hitachi D-6000, Merck USA) using a C18 column (Marchery-Nagel, cc125/4, Nucleosil 100-5, protect 1). IDE was the commercially available recombinant rat insulin degrading enzyme from Calbiochem (Merck Biosciences, San Diego, California, USA). The extracellular domain of human neutral endopeptidase (neprilysin, huNEP) was expressed in Pichia pastoris and purified as previously described (Dale, G. E.; D'Arcy, B.; Yuvaniyama, C.; Wipf, B.; Oefner, C.; D'Arcy, A *Acta Crystallogr D Biol Crystallorg* (2000), 56: 894-897). All experiments were carried out in assay buffer (50 mM Hepes, 100 mM NaCl, 0.05% BSA, pH 7.4) with a total assay volume of 40 µl.

### Chymase

For the chymase a substrate was chosen containing the 6 amino acid peptide CAAPFW-amide (derived from the Chymase substrate succinyl-Ala-Ala-Pro-Phe-p-Nitroanilide (Lockhart BE, et al., "Recombinant human mast-cell chymase: an improved procedure for expression in Pichia pastoris and purification of the highly active enzyme." *Biotechnol Appl Biochem.* published as immediate publication 26 May 2004 as manuscript BA20040074)). The peptide was synthesized with a purity of 95% from Biosyntan GmbH, Berlin (DE). MR121-maleimide (provided by Roche Diagnostics, Penzberg) was coupled as described above to the cystein of the peptide and purified on an analytical HPLC (Merck Hitachi D-6000, Merck USA) using a C18 column (Marchery-Nagel, cc125/4, Nucleosil 100-5, protect 1). Chymase purified form human skin mast cells was obtained from Calbiochem (Merck Biosciences, San Diego, California, USA). The assay buffer was 0.1 M Hepes, 0.02% Na-azid, 0.01% Triton X-100 and 0.1 mg/ml Heparin at pH 7.4.

### Instrumentation

All measurements were performed in 384 well microtiter plates (Costar ref. 3640, clear, non-binding surface). For IDE the total assay volume was 40 µl, for Chymase 50 µl. For the kinetic readout a Zeiss plate::vision reader was used with excitation at 630 nm (bandpass filter: 630(50) nm) and emission at 695 nm (bandpass filter: 695(55) nm) at a constant temperature of 30°C.

### Results

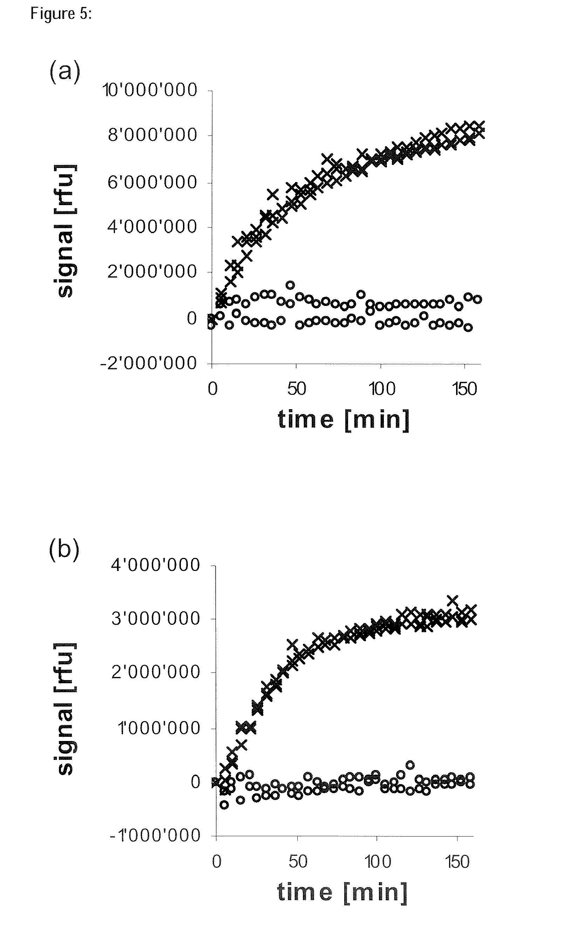

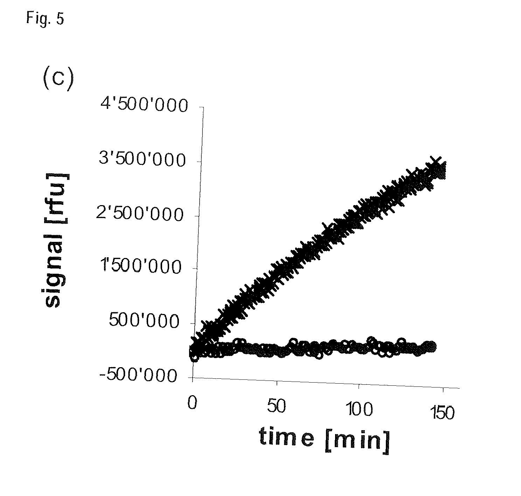

Figure 5 shows kinetic measurements of the enzymatic reactions with 10 nM IDE (a), 1 nM NEP (b) and 5 nM Chymase (c). The concentrations of the respective substrates were 10 µM (IDE), 3 µM (NEP) and 20 µM (Chymase). The readout was started immediately after initiation of the reaction by the addition of the enzyme to the substrate. The MR121 fluorescence was measured for approximately three hours every five minutes (for IDE and NEP) and every minute (for Chymase), respectively.

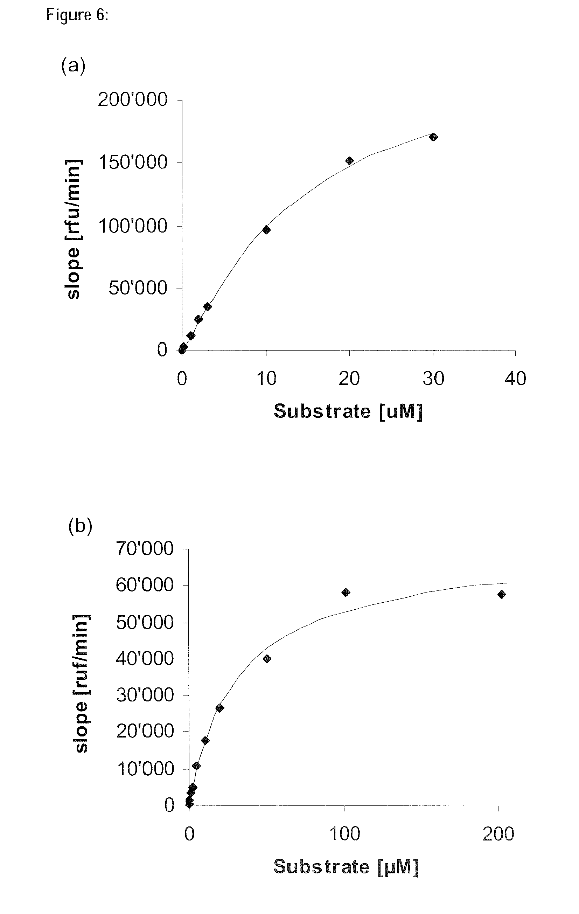

To determine Km and Vmax kinetic measurements with 10 nM IDE and different substrate concentrations ranging from 30 nM to 30 µM were carried out. Because of light absorption at high concentrations of MR121 only 0.1% of the total substrate was labelled with the fluorophore. The readout was started immediately after the reaction was initiated by the addition of 20 µl enzyme to 20 µl substrate and measured for approximately three hours every five minutes. Km and Vmax were calculated from the linear slope of the enzyme kinetic as a function of the total substrate concentration (Fig. 6a). The measurement resulted in Km = 14 µM and Vmax = 242'000 rfu/min. A similar measurement was done for NEP (data not shown) giving Km=1.4 µM and Vmax = 78'000 rfu/min.

For Chymase Km and Vmax were determined with substrate concentrations ranging from 100 nM to 200 µM (Fig. 6b). Here the ratio of labelled to unlabeled substrate was 1:100. A fit of the calculated slopes resulted in Km = 33 µM and Vmax = 71'200 rfu/min.

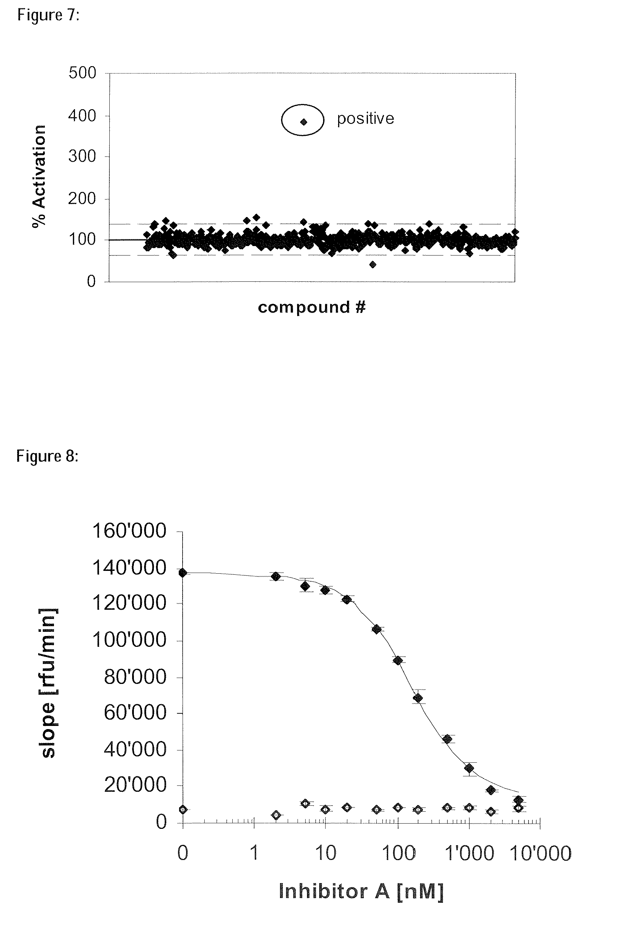

A screen of a small subset of our library was carried out at 20 µM candidate compound, 10 nM IDE and 100 nM substrate (100% MR121 labelled). The compounds were diluted by adding 62.5 µl assay buffer to 2µl of a 5mM DMSO stock solution. 5 µl of the resulting compound solution were transferred to the assay plate containing 20 µl substrate. With the addition of 15 µl IDE the reaction was started and measurements of the MR121 fluorescence were done every two minutes for one hour. Figure 7 shows the activation of IDE by the test compounds where 100% is the enzyme activity without compound. One clearly positive compound was identified, activating IDE approximately by a factor of four. No false positives due to interference of background fluorescence of the compounds were detected proving this assay as robust and sensitive.

Figure 8 shows a dose response curve for a Chymase inhibitor. 5 µl of inhibitor A was added to 25 µl substrate (1 µM final concentration with a ratio labeled:unlabeled substrate = 1:10). The reaction was started by the addition of 20 µl enzyme (5 nM final concentration). Slopes were calculated in the linear range of the kinetic and plotted against the concentration of inhibitor A A calculation of the IC50 resulted in 157 nM.

**A)**

| | | IDE | |
|---|---|---|---|
| Order | Fragment | mass calculated | mass observed |
| 1 | 1-42 | 4512.3 | 4512.6 |
| 2 | 1-19 | 2314.1 | 2314.0 |
| 3 | 1-20 | 2461.2 | 2460.9 |
| 4 | 1-18 | 2167.0 | 2166.8 |
| | 1-13 | 1561.7 | 1561.5 |
| 5 | 1-14 | 1698.7 | 1698.6 |
| 6 | 15-28 | 1581.8 | 1581.9 |

**B)**

| | | NEP | |
|---|---|---|---|
| Order | Fragment | mass calculated | mass observed |
| 1 | 1-42 | 4512.3 | 4512.6 |
| 2 | 1-17 | 2068.0 | 2067.9 |
| | 1-19 | 2314.1 | 2314.1 |
| 3 | 1-16 | 1954.9 | 1954.9 |
| 4 | 1-9 | 1033.4 | 1033.4 |

Table 2: Proteolytic fragments of Abetal-42, following IDE (A) and NEP (B) digestion identified by mass spectrometry. All masses are determined as single charged monoisotopic masses (MH⁺). Order of appearance of the fragments was determined by relative quantitative HPLC analysis.

## Claims

1. A method for identifying activators or inhibitors of enzyme activity comprising
a) contacting a enzyme with (i) a compound of formula F - P - Q
- wherein P represents a peptide comprising at least one cleavage site of the enzyme,
- wherein F represents a fluorophore which is attached to at least one amino acid residue of P,
- wherein Q represents a compound being able to statically quench the fluorophore F, and being attached to at least to one amino acid residue of P, and
- whereby F and Q are attached to amino acid residues on different sites of the enzyme cleavage site of P,
and (ii) a compound of interest, and
b) measuring the fluorescent emission,
c) comparing the resulted fluorescence emission of b) with a control fluorescence emission.

2. A method according to claim 1 wherein the fluorophore F is an oxazine derivative capable of coupling of the general formula I 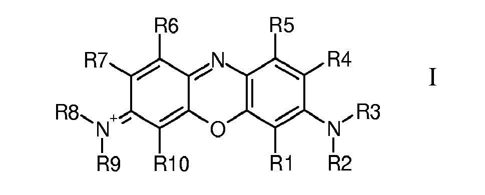 wherein R1, R4, R5, R6, R7 and R10 are each hydrogen, an alkyl of from 1 to 10 carbon atoms, an alkoxy of 1 to 10 carbon atoms, hydroxy, halogen, carboxyl, sulfonyl or amino;
R2 and R3 are each hydrogen, an alkyl of 1 to 10 carbon atoms , alkoxy of 1 to 10 carbon atoms, polyoxyhydrocarbyl, phenyl, phenylalkyl which can be substituted by hydroxy, halogen, sulfonyl, carboxy, amino, alkoxycarbonyl, R2 and R3 optionally substituted by a substituent selected from the group consisting of hydroxyl, suflonyl, carboxyl amino and alkoxycarbonyl, wherein R2 and R1 or R3 and R4 can form a saturated or unsaturated C2 or C3 bridge or R2 and R3 can form a saturated or unsaturated C4 or C5 bridge;
R8, R9 are each hydrogen, an alkyl of 1 to 10 carbon atoms, an alkoxy of 1 to 10 carbon atoms, polyoxyhydrocarbyl, phenyl or phenylalkyl, R8 and R9 optionally substituted by a substituent selected from the group consisting of hydroxy, halogen, sulfonyl, carboxyl, amino, alkoxycarbonyl in which R8 and R7 or R9 and R10 can form a saturated or unsaturated C2 or C3 bridge or R8 and R9 can form a saturated or unsaturated C4 or C5 bridge;
wherein at least one of R2, R3, R8 or R9 is a non-bridge forming residue which is substituted by an activated group capable of coupling or by a group that can be activated to couple,
and at least one of the residues R2, R3, R8 or R9 represents a bridge-forming residue which optionally can be substituted by alkyl.

3. A method according to claims 2 wherein F is a oxazine derivate of formula I in which
R1, R5, R6, R10 are each a hydrogen,
R2 is an ethyl,
R3 and R4 form a saturated C3 bridge (C3H6),
R7 and R8 form a saturated C3 bridge (C3H6),
R9 is a carboxyl (C4H6O2).

4. A method according to any one of the claims 1 to 3 wherein P is an amyloid peptide or a fragment thereof.

5. A method according to any one of the claims 1 to 3, wherein the peptide is Abeta(1-42) or a fragment thereof.

6. A method according to any one of the claims 1 to 3 wherein the peptide has a peptide sequence of the formula: Lys-Leu-Val-Phe-Phe-Ala-Glu-Asp.

7. A method according to any one of the claims 1 to 3 wherein the peptide P has the peptide sequence of the formula: Ala-Ala-Pro-Phe

8. A method according to any one of claims 1 to 7, wherein Q is Tryptophan.

9. A compound of formula F - P - Q
- wherein P represents a peptide comprising at least one cleavage site of the enzyme,
- wherein F represents a fluorophore which is attached to at least one amino acid residue of P,
- wherein Q represents a compound being able to statically quench the fluorophore F, and being attached to at least to one amino acid residue of P, and
- whereby F and Q are attached to amino acid residues on different sides of the enzyme cleavage site of P.

10. A compound according to claim 9 wherein F is an oxazine derivative capable of coupling of the general formula I 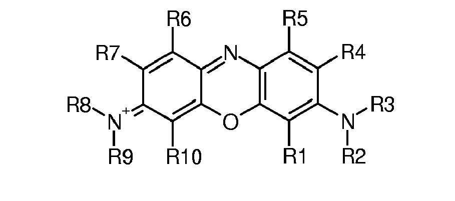 wherein R1, R4, R5, R6, R7 and R10 are each hydrogen, an alkyl of from 1 to 10 carbon atoms, an alkoxy of 1 to 10 carbon atoms, hydroxy, halogen, carboxyl, sulfonyl or amino;
R2 and R3 are each hydrogen, an alkyl of 1 to 10 carbon atoms , alkoxy of 1 to 10 carbon atoms, polyoxyhydrocarbyl, phenyl, phenylalkyl which can be substituted by hydroxy, halogen, sulfonyl, carboxy, amino, alkoxycarbonyl, R2 and R3 optionally substituted by a substituent selected from the group consisting of hydroxyl, suflonyl, carboxyl amino and alkoxycarbonyl, wherein R2 and R1 or R3 and R4 can form a saturated or unsaturated C2 or C3 bridge or R2 and R3 can form a saturated or unsaturated C4 or C5 bridge;
R8, R9 are each hydrogen, an alkyl of 1 to 10 carbon atoms, an alkoxy of 1 to 10 carbon atoms, polyoxyhydrocarbyl, phenyl or phenylalkyl, R8 and R9 optionally substituted by a substituent selected from the group consisting of hydroxy, halogen, sulfonyl, carboxyl, amino, alkoxycarbonyl in which R8 and R7 or R9 and R10 can form a saturated or unsaturated C2 or C3 bridge or R8 and R9 can form a saturated or unsaturated C4 or C5 bridge;
wherein at least one of R2, R3, R8 or R9 is a non-bridge forming residue which is substituted by an activated group capable of coupling or by a group that can be activated to couple,
and at least one of the residues R2, R3, R8 or R9 represents a bridge-forming residue which optionally can be substituted by alkyl.

11. A compound according to claim 10 wherein F is a oxazine derivate of formula I in which
R1, R5, R6, R10 are each a hydrogen,
R2 is an ethyl,
R3 and R4 form a saturated C3 bridge (C3H6),
R7 and R8 form a saturated C3 bridge (C3H6),
R9 is a carboxyl (C4H6O2).

12. A compound according to any one of the claims 9 to 11 wherein P is an amyloid peptide or a fragment thereof.

13. A compound according to any one of the claims 9 to 11 wherein P is Abeta(1-42) or a fragment thereof.

14. A compound according to any one of the claims 9 to 11 wherein P has a peptide sequence of the formula Lys-Leu-Val-Phe-Phe-Ala-Glu-Asp.

15. A compound according to any one of the claims 9 to 11 wherein P has a peptide sequence of the formula Ala-Ala-Pro-Phe.

16. A compound according to any one of claims 9 to 15, wherein Q is Tryptophan.

17. A compound according claim 9 wherein the compound has the formula:
MR121-linker- Lys-Leu-Val-Phe-Phe-Ala-Glu-Asp -Trp.

18. An activator or/and inhibitor identified by the method according to any one of the claims 1 to 8.

19. Suramin as an activator of insulin degrading enzyme (IDE).

20. Suramin for preparation of a medicament for treating Alzheimer disease.

21. A pharmaceutical composition comprising a soluble activator or inhibitor of claims 18 or 19 and a compatible pharmaceutically acceptable carrier material.
